# EUROPEAN PATENT SPECIFICATION

(11) **EP 1 876 192 B1**
(45) Date of publication and mention of the grant of the patent: **05.08.2009**
(21) Application number: 07252333.5
(22) Date of filing: 08.06.2007
(51) Int. Cl.: C08F 265/06, A61Q 5/06, A61K 8/81

(54) **Multistage polymer composition and method of use**
Mehrstufige Polymerzusammensetzung und Verfahren zu dessen Verwendung
Composition polymère à phases multiples et son procédé d'utilisation

(30) Priority: 13.06.2006 US 813073 P
(43) Date of publication of application: 09.01.2008
(73) Proprietor: Rohm and Haas Company, Philadelphia, PA 19106-2399 (US)
(72) Inventor: Keller, Kathleen Virgina, Horsham PA 19044 (US); Kosto, Kim Bryan, Maple Glen PA 19002 (US); Nakatani, Alan Isamu., Lansdale PA 19446 (US); Wang, Miao, Schwenksville PA 19473 (US); Zeng, Fanwen, Belle Mead NJ 08502 (US)
(74) Representative: Buckley, Guy Julian

(56) References cited:
- WO-A-01/51018
- US-A- 5 859 112
- US-A- 5 929 173
- US-A- 5 990 228
- US-A1- 2003 053 976

## Description

Many hair styling compositions contain one or more polymer. The polymer or polymers are thought to contribute to one or more of a variety of desirable properties that relate to hair that has been styled using these compositions. These desirable properties of such polymers include, for example, durability, resistance to high humidity, low tackiness, and good hold. For example, US Patent Application Publication 2004/0096474 discloses hair styling compositions that contain two different polymers and a cosmetically acceptable solvent. It is desired to provide compositions that improve on the desirable properties of the styled hair, including, for example, resistance to high humidity.

Independently, it is desired to improve one or more properties that relate to the manufacture of a hair styling composition. For example, in some cases it is desired to produce polymer in the form of a powder, which can then be added to a hair styling composition. In such cases, it is desirable that the powder be free flowing.

Also independently, it is desired to improve one or more properties that relate to the properties of the hair styling composition itself, prior to applying the hair styling composition to hair. For example, in some cases, the hair styling composition is a liquid, and it is desired to provide liquid hair styling compositions with optimized viscosity. For example, in some cases it is desired to provide liquid hair styling compositions with reduced viscosity.

In one aspect of the present invention, there is provided a multistage polymer that comprises
(a) at least one soft polymer having glass transition temperature of 40°C or lower, and
(b) at least one hard polymer having glass transition temperature higher than 40°C, wherein the glass transition temperature of said hard polymer is at least 10°C higher than the glass transition temperature of said soft polymer,

wherein said hard polymer comprises monomer units, by weight based on the weight of said hard polymer, of
(i) 5% to 50% unsubstituted alkyl esters of acrylic acid,
(ii) 10% to 75% unsubstituted alkyl esters of methacrylic acid,
(iii) 2% to 50% hydroxyalkyl esters of acrylic acid or of methacrylic acid, and
(iv) 10% to 30% acid functional monomer units
wherein the weight ratio of said hard polymer to said soft polymer is from 1.01:1 1 to 100:1, and wherein said multistage polymer, after exposure to liquid water followed by drying at temperatures below 100°C, shows maximum thermal transition temperature in an atmosphere of 0% relative humidity that differs by 20°C or less from the maximum thermal transition temperature in an atmosphere of 75% relative humidity.

In some embodiments, a composition of the present invention is used in a hair styling composition. As used herein, the term "hair styling composition" means a pump or aerosol hair spray, styling gel, styling glaze, spray foam, styling cream, styling wax, styling lotion, liquid foam, spray gel, pomade, blow-dry lotion, curl activator, or mousse that is used on hair to hold the hair in a particular shape or configuration. In some embodiments, the hair styling composition in the present invention is a hair spray. The term "hair" means natural human hair, animal hair, artificial hair, and wigs or hairpieces comprising hair.

A "polymer," as used herein and as defined by FW Billmeyer, JR. in Textbook of Polymer Science, second edition, 1971, is a relatively large molecule made up of the reaction products of smaller chemical repeat units. Polymers may have structures that are linear, branched, star shaped, looped, hyperbranched, crosslinked, or a combination thereof; polymers may have a single type of repeat unit ("homopolymers") or they may have more than one type of repeat unit ("copolymers"). Copolymers may have the various types of repeat units arranged randomly, in sequence, in blocks, in other arrangements, or in any mixture or combination thereof.

"Polymerizing" herein means the reacting of monomers to form polymer.

Polymerizing may be performed by any type of polymerization process, including, for example, emulsion polymerization, microemulsion polymerization, solution polymerization, bulk polymerization, suspension polymerization, or combinations thereof. In some cases, emulsion polymerization is performed using aqueous emulsion, and the product is an aqueous polymer latex.

Polymer molecular weights can be measured by standard methods such as, for example, size exclusion chromatography or intrinsic viscosity. Generally, polymers have weight-average molecular weight (Mw) of 1,000 or more. Polymers may have extremely high Mw; some polymers have Mw above 1,000,000; typical polymers have Mw of 1,000,000 or less. Some polymers are crosslinked, and crosslinked polymers are considered to have infinite Mw.

Molecules that can react with each other to form the repeat units of an oligomer or a polymer are known herein as "monomers." Typical monomers have molecular weight of less than 400. Among the monomers useful in the present invention are molecules, for example, that have at least one carbon-carbon double bond. Among such monomers are, for example, vinyl monomers, which are molecules that have at least one vinyl group (i.e., CH2=CR-, where R is a hydrogen, a halogen, an alkyl group, a substituted alkyl group, or another substituted or unsubstituted organic group). Some suitable vinyl monomers include, for example, styrene, substituted styrenes, dienes, ethylene, ethylene derivatives, and mixtures thereof. Ethylene derivatives include, for example, unsubstituted or substituted versions of the following: vinyl acetate, acrylonitrile, (meth)acrylic acids, (meth)acrylates, (meth)acrylamides, vinyl chloride, halogenated alkenes, and mixtures thereof. As used herein, "(meth)acrylic" means acrylic or methacrylic; "(meth)acrylate" means acrylate or methacrylate; and "(meth)acrylamide" means acrylamide or methacrylamide. In some embodiments, "substituted" monomers include, for example, monomers with more than one carbon-carbon double bond, monomers with hydroxyl groups, monomers with other functional groups, and monomers with combinations of functional groups.

A polymer that is made by polymerizing a certain monomer, either alone or with other monomers, is said herein to include that monomer as a monomer unit.

In some embodiments, the present invention involves the use of one or more chain transfer agent. Chain transfer agents are compounds capable of participating in a chain transfer reaction during radical polymerization of monomer. Some suitable chain transfer agents are, for example, halomethanes, disulfides, thiols (also called mercaptans), and metal complexes. Also suitable as chain transfer agents are various other compounds that have at least one readily abstractable hydrogen atom. Mixtures of suitable chain transfer agents are also suitable. Suitable thiols include, for example, aryl thiols, alkyl thiols, alkyl dithiols, mercaptoalkanols, and alkyl esters of thioalkyl carboxylic acids. Some suitable thiols are, for example, benzene thiol, dodecyl mercaptans, hexanethiol, butanethiol, butyl 3-mercaptopropionate, ethyl 3-mercaptopropionate, butyl mercaptoacetate, 1,6-hexanedithiol, 4-mercapo-2-butanol, 4-mercapto-1-butanol, and 2-mercapto-ethanol. Suitable halomethanes include, for example, chloroform, tetrabromomethane, tetrachloromethane, and bromotrichloromethane. Some suitable disulfides include, for example, dialkyldisulfides (such as, for example diethyldisulfide), dialkylaryldisulfides (such as, for example, dibenzyldisulfide), and diaryldisulfides (such as, for example, diphenyldisulfide).

As used herein, a composition is "aqueous" if it contains 25% or more water by weight based on the weight of the composition. Some aqueous compositions contain 40% or more; or 50% or more; water by weight, based on the weight of the composition. In some aqueous compositions, water forms a continuous medium, and one or more other substance is dissolved or dispersed in the water. In aqueous compositions in which water forms a continuous medium, the water may or may not be mixed with one or more additional solvents that are miscible with water. In some aqueous compositions in which water forms a continuous medium, the continuous medium contains 30% or more water; or 50% or more water; or 75% or more water; or 90% or more; water, by weight based on the weight of the continuous medium.

In some embodiments, the practice of the present invention involves the use of an aqueous polymer latex, which is an aqueous composition in which discrete polymer particles are dispersed in a continuous medium. Typically, the polymer is made by a process of emulsion polymerization. Independently, typically, the continuous medium is 75% or more water, by weight based on the weight of the continuous medium. In some latexes, the polymer particles have mean diameter of 10 nm or larger, or 30 nm or larger, or 100 nm or larger. Independently, in some latexes, the polymer particles have mean diameter of 2,000 nm or smaller; or 1,000 nm or smaller, or 500 nm or smaller. In some cases polymer latex has polymer solids of 60% or less or 50% or less by weight based on the weight of the latex. In some cases, a polymer latex may have polymer solids of 25% or more; or 35% or more; or 45% or more; by weight based on the weight of the latex.

As used herein, the glass transition temperature (Tg) of a polymer is measured by differential scanning calorimetry (DSC). A polymer may have more than one Tg. Measurement of Tg is normally performed on polymer samples that have been thoroughly dried to remove water. Such thoroughly dried samples, if they contain water, contain water in amounts so small that they do not affect the measurement of Tg. Measurement of Tg is also normally done while the sample of polymer is kept in a dry atmosphere. Herein, if a polymer is described as having a certain Tg, without specifying any particular degree of drying or any particular relative humidity, it is meant that the polymer shows that Tg when tested after it has been thoroughly dried and then measured under dry atmosphere (i.e., 0% relative humidity).

In the practice of the present invention, the multistage polymer has been exposed to water followed by drying at temperature less than 100°C. Such drying may take place, for example, in some embodiments in which the multistage polymer is used in a hair spray. It is contemplated that multistage polymer may be exposed to water in a variety of ways. By "exposed to water" it is meant herein that the multistage polymer is in contact with liquid water in a manner that allows the multistage polymer to acquire an equilibrium amount of water. Multistage polymer may be exposed to water by being a component in an aqueous latex (for example, when the multistage polymer is made by emulsion polymerization), by being dissolved in a solvent that contains water, by soaking in water, by other means, or by any combination thereof.

To understand the behavior of multistage polymer that has been dried at temperature less than 100°C, it is sometimes useful obtain a sample of a multistage polymer that has been exposed to water, to dry that sample at temperature less than 100°C, and to test the resulting sample by DSC. In some cases, thermal transition temperatures can be identified by the normal methods that are usually used to identify glass transition temperatures of polymers.

Samples of multistage polymer that are dried at temperature less than 100°C may or may not be thoroughly dried. That is, they may show thermal transition temperatures in DSC measurement that are affected by the presence of water in the samples. Such transition temperatures, therefore, may not be the standard glass transition temperatures of the multistage polymer, as defined herein above. When the thermal transition temperature or temperatures in such a sample are less than the standard glass transition temperature or temperatures of the multistage polymer, the sample is said to be hydroplasticized.

DSC measurements can be made on polymer samples, whether they are thoroughly dried or not. Independently, DSC measurements can be made in the standard way using an atmosphere of 0% relative humidity, or DSC measurements can be made in an atmosphere with higher relative humidity, for example at 75% relative humidity. It is contemplated that DSC measurements made at non-zero relative humidity could help predict whether a polymer sample is likely to change its properties when it put into use, for example as a hair fixative, and the ambient humidity changes.

The multistage polymers of the present invention have the useful property that, when dried at temperature less than 100°C, whether or not the resulting sample is hydroplasticized, the maximum thermal transition temperature (i.e., the highest thermal transition temperature, if more than one thermal transition temperature is observed) of the resulting sample does not change very much if the atmosphere of the DSC measurement changes from 0% relative humidity to 75% relative humidity. In general, when a multistage polymer of the present invention is dried at temperature less than 100°C and then measured by DSC at 0% relative humidity and at 75% relative humidity, the maximum thermal transition temperature observed at 0% relative humidity is different from the maximum thermal transition temperature observed at 75% relative humidity by 20°C or less; or by 10°C or less; or by 5°C or less.

Independently, in some embodiments, when a multistage polymer of the present invention is dried at temperature less than 100°C and then measured by DSC at 0% relative humidity, the maximum thermal transition temperature observed at 0% relative humidity is less than the maximum glass transition temperature of the multistage polymer by 10°C or more; or 20°C or more; or 30°C or more.

In some embodiments, when a multistage polymer of the present invention is dried at temperature less than 100°C, whether or not the resulting sample is hydroplasticized, the minimum thermal transition temperature (i.e., the lowest thermal transition temperature, if more than one thermal transition temperature is observed) of the resulting sample does change if the atmosphere of the DSC measurement changes from 0% relative humidity to 75% relative humidity. In some embodiments, when a multistage polymer of the present invention is dried at temperature less than 100°C and then measured by DSC at 0% relative humidity and at 75% relative humidity, the minimum thermal transition temperature observed at 0% relative humidity is different from the minimum thermal transition temperature observed at 75% relative humidity by 10°C or more; or by 20°C or more; or by 30°C or more.

Independently, in some embodiments, when a multistage polymer of the present invention is dried at temperature less than 100°C and then measured by DSC at 0% relative humidity, the minimum thermal transition temperature observed at 0% relative humidity is different from the minimum glass transition temperature of the multistage polymer by 20°C or less; or 10°C or less.

As used herein, a "multistage" polymer is a polymer that is made in more than one polymerization stage. A polymerization stage is a process in which polymerization takes place and then effectively ends. That is, at the end of a polymerization stage, little or no monomer is present (i.e., the amount of monomer is 10% or less, or 5% or less, or 2% or less, by weight based on the weight of polymer produced by that polymerization stage), and the rate of polymerization is negligible or zero. In a multistage polymerization process, after the first stage is ended, at least one further stage is conducted in the presence of the polymer made by the previous stage. Optionally, one or more additional polymerization stage may be conducted; each stage is performed after the previous polymerization stage has effectively ended.

In some embodiments, the multistage polymer is made by a multistage emulsion polymerization process. That is, a first polymer is made by a process of emulsion polymerization (the first stage). Then, in the presence of the polymer produced by the first stage, a second emulsion polymerization process (the second stage) is conducted. In some embodiments, the composition of the polymer made during the second stage is different from the composition of the polymer made during the first stage. In some embodiments, some or all of the polymer made in the first stage is left in place in the vessel in which the first stage was conducted, and the second stage is conducted in the same vessel. In some embodiments, the polymer made in the first stage is removed and placed in a new container, with or without dilution by water, and the second stage is performed in the new container. After the second stage, further stages may or may not be conducted.

In some embodiments, the first stage is an emulsion polymerization process that produces a polymer latex. In some of such embodiments, when a second stage is conducted, most or all of the polymer produced in the second stage is formed on, in, or attached to the latex particles made in the first stage. Thus, the result is a latex in which most or all of the particles each contain polymer from the first stage and polymer from the second stage. If subsequent stages are conducted, in some cases the polymer from each subsequent stage will form on, in, or attached to the particles formed in the previous stage.

The present invention involves the use of at least one soft polymer. A soft polymer is a polymer with a Tg of 40°C or lower. In some embodiments, a soft polymer is used that has a Tg of -50°C or higher; or -25°C or higher; or 0°C or higher; or 25°C or higher. In some embodiments, at least one soft polymer is used that has only one glass transition temperature.

The present invention involves the use of at least one hard polymer. A hard polymer is a polymer with a Tg of higher than 40°C. In some embodiments, a hard polymer is used that has a Tg of 60°C or higher; or 80°C or higher. Independently, in some embodiments, a hard polymer is used that has a Tg of 200°C or lower; or 150°C or lower; or 120°C or lower. In some embodiments, at least one hard polymer is used that has only one glass transition temperature.

In the practice of the present invention, at least one hard polymer and at least one soft polymer are used, chosen so that the Tg of the hard polymer is at least 10°C higher than the Tg of the soft polymer. In some embodiments, the Tg of the hard polymer is at least 20°C higher, or at least 30°C higher, or at least 40°C higher, or at least 50°C higher, than the Tg of the soft polymer.

In the practice of the present invention, at least one hard polymer and at least one soft polymer are used in amounts such that the weight ratio of hard polymer to soft polymer is from 1.01:1 1 to 100:1. In some embodiments, the weight ratio of hard polymer to soft polymer is 1.05:1 or higher (i.e., the weight ratio is X: 1, where X is 1.05 or higher); or 1.1:1 or higher; or 1.2:1 or higher; or 1.3:1 or higher; or 1.4:1 or higher. In some embodiments, the weight ratio of hard polymer to soft polymer is 4:1 or lower; or 3:1 or lower; or 2:1 or lower; or 1.6:1 or lower.

In some embodiments, a thoroughly dried film made from the multistage polymer of the present invention shows at least two distinct glass transition temperatures. It is contemplated that one glass transition temperature is due to a soft polymer and a separate glass transition temperature is due to a hard polymer. The existence of separate glass transition temperatures may be observed by any measurement technique, including, for example, differential scanning calorimetry or dynamic mechanical analysis. For example, the appearance of separate peaks in the tandelta curve versus temperature (from dynamic mechanical analysis) is considered evidence of the existence of separate glass transition temperatures.

The soft polymer of the present invention may have any composition. In some embodiments, no soft polymer is used that is a polyester. In some embodiments, no soft polymer is used that has an ester linkage as part of the polymer backbone. Independently, in some embodiments, at least one soft polymer is used that is a vinyl polymer. In some embodiments, no soft polymer is used that is not a vinyl polymer.

Vinyl polymers are polymers that have monomer units that are 50% or more vinyl monomers by weight, based on the weight of the polymer. Some vinyl polymers have 75% or more, or 80% or more, or 90% or more; or 96% or more; vinyl monomer units by weight, based on the weight of the polymer.

Independently, in some embodiments, at least one soft polymer is used that is an acrylic polymer.

Acrylic polymers are polymers that have monomer units that are 50% or more acrylic monomers by weight, based on the weight of the polymer. Some acrylic polymers have 75% or more, or 80% or more, or 90% or more acrylic monomer units by weight, based on the weight of the polymer. Acrylic monomers include acrylic acid, methacrylic acid, esters thereof (i.e., "acrylic esters") and amides thereof (i.e., "acrylic amides"). Acrylic esters may be substituted or unsubstituted. Acrylic esters include, for example, C1 to C22 alkyl (straight, branched, or cyclic) esters, which may be substituted or unsubstituted. In some cases, acrylic polymers include copolymerized monomer units of monomers that are vinyl monomers other than acrylic monomers. Vinyl monomers other than acrylic include, for example, styrene, substituted styrenes, vinyl esters of organic acids, N-vinyl compounds, dienes, maleic acid, maleic anhydride, other unsaturated dicarboxylic acids or their anhydrides, and mixtures thereof.

In some embodiments, a soft polymer is used that contains monomer units that are unsubstituted alkyl esters of acrylic acid, including, for example, esters in which the alkyl group has one or more carbon atoms, or two or more carbon atoms. Independently, in some embodiments, unsubstituted alkyl esters of acrylic acid are used in which the alkyl group has 22 or fewer carbon atoms; or 8 or fewer carbon atoms; or 6 or fewer carbon atoms; or 4 or fewer carbon atoms. In some embodiments, a soft polymer is used that contains monomer units of two or more different unsubstituted alkyl esters of acrylic acid. In some embodiments in which unsubstituted alkyl esters of acrylic acid are present in the soft polymer, the amount of unsubstituted alkyl esters of acrylic acid is, for example, 40% or more; or 50% or more; or 60% or more by weight, based on the weight of the soft polymer. Independently, in some embodiments in which unsubstituted alkyl esters of acrylic acid are present in the soft polymer, the amount of unsubstituted alkyl esters of acrylic acid is, for example, 95% or less; or 85% or less; or 80% or less by weight, based on the weight of the soft polymer.

Independently, in some embodiments, a soft polymer is used that contains one or more hydroxyalkyl ester of acrylic acid or methacrylic acid. The alkyl group in such a hydroxyalkyl ester may have one or more carbon atoms, or two or more carbon atoms. Independently, the alkyl group in such a hydroxyalkyl ester may have 8 or fewer carbon atoms; or 4 or fewer carbon atoms. In some embodiments, at least one hydroxyalkyl ester of methacrylic acid is used. In some embodiments in which monomer units of hydroxyalkyl ester of acrylic acid or methacrylic acid are present in the soft polymer, the amount of such monomer units is, for example, 5% or more, or 10% or more by weight based on the weight of soft polymer. Independently, in some embodiments in which monomer units of hydroxyalkyl ester of acrylic acid or methacrylic acid are present in the soft polymer, the amount of such monomer units is, for example, 50% or less; or 30% or less; or 20% or less by weight, based on the weight of soft polymer.

Independently, in some embodiments, a soft polymer is used that contains one or more acid-functional monomer units. Acid functional monomer units may be, for example, polymerized units of acrylic acid, methacrylic acid, itaconic acid, maleic acid, any other unsaturated carboxyl compound, or mixtures thereof. In some embodiments, monomer units of acrylic acid or methacrylic acid or a mixture thereof are used. In some embodiments, monomer units of methacrylic acid are used. In some embodiments in which acid functional monomer units are present in the soft polymer, the amount of acid functional monomer units is, for example, 1% or more; or 2% or more; or 5% or more; or 10% or more; by weight based on the weight of soft polymer. Independently, in some embodiments in which acid functional monomer units are present, the amount of acid functional monomer units is, for example, 30% or less; or 20% or less; by weight, based on the weight of soft polymer.

In some embodiments, a soft polymer is made by polymerization of a mixture that contains at least one monomer and at least one chain transfer agent. When a chain transfer agent is used in making a soft polymer, the amount of chain transfer agent is, in some embodiments, 0.1% or more; or 0.2% or more; or 0.5% or more; or 0.9% or more; by weight based on the weight of all monomers in the mixture used for making that soft polymer. Independently, when a chain transfer agent is used in making a soft polymer, the amount of chain transfer agent is, in some embodiments, 3% or less; or 2% or less; or 1.5% or less; by weight based on the weight of all monomers in the mixture used for making that soft polymer.

The hard polymer of the present invention may have any composition.

In some embodiments in which unsubstituted alkyl esters of acrylic acid are present in the hard polymer, the amount of unsubstituted alkyl esters of acrylic acid is, for example, 10% or more; or 20% or more by weight, based on the weight of the hard polymer. Independently, in some embodiments in which unsubstituted alkyl esters of acrylic acid are present in the hard polymer, the amount of unsubstituted alkyl esters of acrylic acid is, for example, 40% or less; or 30% or less by weight, based on the weight of the hard polymer.

In some embodiments, a hard polymer is used that contains monomer units that are unsubstituted alkyl esters of methacrylic acid in which the alkyl group has one or more carbon atoms. Independently, in some embodiments, unsubstituted alkyl esters of methacrylic acid are used in which the alkyl group has 6 or fewer carbon atoms; or 4 or fewer carbon atoms; or 2 or fewer carbon atoms. In some embodiments, a hard polymer is used that contains monomer units of methyl methacrylate. Independently, in some embodiments in which unsubstituted alkyl esters of methacrylic acid are present in the hard polymer, the amount of unsubstituted alkyl esters of methacrylic acid is, for example, 20% or more; or 40% or more by weight, based on the weight of the hard polymer. Independently, in some embodiments in which unsubstituted alkyl esters of methacrylic acid are present in the hard polymer, the amount of unsubstituted alkyl esters of acrylic acid is, for example or 65% or less; or 55% or less by weight, based on the weight of the hard polymer.

In some embodiments in which monomer units of hydroxyalkyl ester of acrylic acid or methacrylic acid are present in the hard polymer, the amount of such monomer units is, for example, 5% or more; or 8% or more; by weight based on the weight of hard polymer. Independently, in some embodiments in which monomer units of hydroxyalkyl ester of acrylic acid or methacrylic acid are present in the hard polymer, the amount of such monomer units is, for example, 25% or less; or 15% or less by weight, based on the weight of hard polymer.

In some embodiments in which acid functional monomer units are present in the hard polymer, the amount of acid functional monomer units is 10% or more and 30% or less; or 20% or less; by weight, based on the weight of hard polymer.

In some embodiments, a hard polymer is made by polymerization of a mixture that contains at least one monomer and at least one chain transfer agent. When a chain transfer agent is used in making a hard polymer, the amount of chain transfer agent is, in some embodiments, 0.05% or more; or 0.1% or more; by weight based on the weight of all monomers in the mixture used for making that hard polymer. Independently, when a chain transfer agent is used in making a hard polymer, the amount of chain transfer agent is, in some embodiments, 0.5% or less; or 0.4% or less; by weight based on the weight of all monomers in the mixture used for making that hard polymer.

In some embodiments, a hard polymer is polymerized in the presence of a soft polymer. Independently, in some embodiments a soft polymer is polymerized in the presence of a hard polymer. In some embodiments, a multistage polymer is made that contains no polymer other than a hard polymer and a soft polymer. Optionally, one or more additional polymers may be polymerized before the soft polymer and the hard polymer, and the hard polymer and soft polymer may be polymerized in the presence of such additional polymer. Independently optionally, one or more additional polymers may be polymerized in between the polymerization of the soft polymer and the hard polymer. Also independently optionally, one or more additional polymers may be polymerized in the presence of both a hard polymer and a soft polymer. Any additional polymer may or may not qualify as a hard polymer or a soft polymer as defined herein.

In some particular embodiments, a multistage polymer is made by first making a soft polymer by emulsion polymerization to produce an aqueous latex of soft polymer particles. In the presence of that latex of soft polymer particles, a hard polymer is polymerized. It is contemplated that, in some embodiments, the hard polymer forms a complete or partial shell around most or all of the soft polymer particles. It is contemplated that such a complete or partial shell can, in some cases, when a powder is made by isolating such an aqueous latex, increase the tendency of the resulting powder to be free flowing.

In some embodiments, a hard polymer is used that has weight-average molecular weight (Mw) of 50,000 or higher; or 70,000 or higher; or 100,000 or higher. Independently, in some embodiments, a hard polymer is used that has Mw of 2,00,000 or lower; or 250,000 or lower; or 200,000 or lower.

In some embodiments, a soft polymer is used that has Mw of 25,000 or higher; or 30,000 or higher; or 40,000 or higher. Independently, in some embodiments, a soft polymer is used that has Mw of 1,000,000 or lower; or 300,000 or lower; or 100,000 or lower.

The Mw can be measured by size exclusion chromatography. In some cases, it is desired to measure the Mw of a polymer of interest that, in the practice of the present invention, will be polymerized in the presence of previous polymer. In such cases, a reasonable estimate of the Mw of the polymer of interest may be obtained by performing a special polymerization for testing purposes; that is, the polymerization process that produces the polymer of interest may be performed as it would be performed in the practice of the present invention, with the exception that the previous polymer is absent during the polymerization of the polymer of interest. The product of this special polymerization can then be measured by size exclusion chromatography to obtain a reasonable estimate of the Mw of the polymer of interest as it will exist in the practice of the present invention.

In some embodiments, a multistage polymer of the present invention is provided in the form of an aqueous latex. Such a latex may be used, for example, by mixing the latex directly with a cosmetically acceptable solvent to form a polymer solution that is suitable for use in hair styling compositions. Cosmetically acceptable solvents include, for example, monoalcohols such as, for example, alcohols containing from 1 to 8 carbon atoms including ethanol, isopropanol, benzyl alcohol, and phenylethyl alcohol; polyalcohols such as, for example, alkylene glycols such as glycerine, ethylene glycol and propylene glycol; glycol ethers such as mono-, di-, and tri-ethylene glycol monoalkyl ethers; ketones, ethers, esters; and mixtures thereof.

For example, a multistage polymer of the present invention in the form of an aqueous latex may be mixed with a water-miscible alcohol and, optionally, with additional water. Independently, in some embodiments, a polymer solution is formed that has polymer solids, by weight based on the weight of solution, of 20% or lower; or 10% or lower; or 7% or lower. Independently, in some embodiments, a polymer solution is formed that has polymer solids, by weight based on the weight of solution, of 1% or higher; or 2% or higher; or 3% or higher. For example, such a latex may be mixed with water and ethanol in amounts chosen to yield a solution that 5% polymer solids by weight based on the weight of the solution and that has a solvent that is a mixture of ethanol and water with a ratio of ethanol to water of 55 to 40. In some embodiments, a suitable water-miscible alcohol is ethanol.

In some embodiments, a multistage polymer of the present invention is provided as a powder. One method, for example, of providing such a powder is to prepare a multistage polymer of the present invention as an aqueous latex and then isolate the multistage polymer (i.e., remove most or all of the water from the aqueous latex). Two common methods of isolation are, for example, spray drying and coagulation.

In some embodiments, a powder that contains one or more multistage polymer of the present invention may be dissolved in a cosmetically acceptable solvent. In such embodiments, the cosmetically acceptable solvent may or may not contain water.

In spray drying, the latex is atomized (i.e., turned into droplets), usually by a wheel or a nozzle, in an atomization chamber. The droplets are thought to lose water through evaporation and become solid particles. Usually, drying gas (normally heated air or heated nitrogen) is forced into the atomization chamber. The temperature of the drying gas is normally regulated to provide a desired temperature of the dry powder. Powder temperature is usually maintained at 80°C or lower; or 65°C or lower; or 55°C or lower. Independently, powder temperature is usually maintained at 20°C or higher; or 30°C or higher; or 40°C or higher.

In spray drying processes, one or more flow aid may or may not be added. Flow aid is a substance, either organic or inorganic, that is added to the powder to improve the powder's ability to flow freely. Flow aid may be added into the atomization chamber as a powder; in some cases, flow aid is supplied as a solid dispersed in water and is spray dried at the same time as the multistage polymer of the present invention. Flow aid desirably has glass transition temperature or melting point higher than the conditions of spray drying. Flow aid normally has mean particle size from 5 nm to 10,000 nm. Flow aid desirably is compatible with hair styling formulations; for example, flow aid is desirably soluble in the solvent to be used in a hair styling formulation but does not significantly raise the solution viscosity. Spray dried powder normally has content of volatile compounds (i.e., compounds including water and other compounds that evaporate from the powder under the same conditions under which water evaporates) of 15% or less; or 10% or less; or 5% or less; or 3% or less; by weight based on the weight of the spray dried powder.

Coagulation is performed by altering the conditions that keep the polymer particles of the latex in a stable dispersion. The latex then becomes unstable, and the polymer solids can be more readily separated from the water. Common coagulation methods involve addition of a coagulant such as, for example, acid or salt. The choice of coagulant and of the concentration of coagulant is determined by the nature of the latex and the method employed to stabilize the latex. Salts used for coagulation include, for example, chlorides. Salts with divalent or trivalent cations are normally considered more effective than salts with monovalent cations. Coagulation normally causes the latex particles to coalesce into a form usually called a slurry. Slurry is normally further treated, using one or more of, for example, addition of further coagulant, raised temperature, addition of flow aid, dewatering (for example, on a vacuum filter belt), centrifuging, squeezing, and drying (for example, in a flash dryer or fluid bed dryer or both). Powder from coagulation process (including drying) normally has content of volatile compounds of 15% or less; or 5% or less; or 2% or less; or 0.5% or less; by weight based on the weight of powder from coagulation process.

In some embodiments, multistage polymer of the present invention is used in the form of a solution in a solvent. As used herein, "solution" includes any composition in which the multistage polymer is dissolved in the solvent, regardless of the type of solvent, and regardless of the viscosity of the solution. In some embodiments (such as, for example, solutions suitable for spraying), the solution is a liquid and thus has relatively low viscosity. In some embodiments, the solution may have much higher viscosity, such as, for example, a gel, lotion, cream, or paste. In some embodiments, the solution may be a foam. In some embodiments, the solution may be a solid, such as, for example, a waxy solid.

Solutions of multistage polymer in a solvent may be obtained by any method. For example, embodiments are contemplated in which a multistage polymer in latex form becomes soluble in water after neutralization. Also contemplated, for example, are embodiments in which multistage polymer in aqueous latex form is treated by addition of a water-soluble solvent to the latex to create a solution of multistage polymer in a solvent that is a mixture of water and the water-soluble solvent. Also contemplated, for example, are embodiments in which multistage polymer is extracted from latex form by treating the latex with a water-insoluble polymer. Also contemplated, for example, are embodiments in which solid multistage polymer is dissolved in solvent.

Independent of the method used for making a solution of multistage polymer in solvent, suitable solvents include, for example, water that is not mixed with other solvents, water mixed with other water-soluble solvent, water-soluble solvent that is not mixed with water, and water-insoluble solvent. Among embodiments in which a solvent is used that is a mixture of water with a water-soluble solvent, in some embodiments the ratio of ethanol to water is 0.25:1 or higher; or 0.54:1 or higher; or 1:1 or higher. Independently, among embodiments in which a solvent is used that is a mixture of water with a water-soluble solvent, in some embodiments the ratio of ethanol to water is 4:1 or lower; or 2.3:1 or lower; or 1.5:1 or lower.

In some embodiments, the practice of the present invention involves an anhydrous solution of a multistage polymer. An anhydrous solution is a solution that contains 5% or less water by weight based on the weight of the solution. In some embodiments, an anhydrous solution is used that has 2% or less; or 1% or less; or 0.5% or less; or 0.2% or less; water by weight based on the weight of the solution.

Also contemplated are embodiments in which a hair styling composition is used in which a multistage polymer is used in latex form. In such embodiments, the continuous medium may be water or a mixture of water and water-soluble solvent.

The polymers utilized in the polymer composition of this invention should be compatible in hair styling compositions. To test the compatibility of the polymers, the polymers are first dissolved in a mutual solvent to form a solution of the polymers. The solvent is evaporated leaving a film. Incompatible polymers will form a cloudy film with poor mechanical properties, including low shear storage modulus at higher temperatures. A characteristic of the polymer compositions of this invention is that when dried, they form flexible, tough films characterized as having a shear storage modulus, G', at 25° C of from 1x10⁹ Pascal ("Pa") to 1x10⁷ Pa and G' at 70° C of from 1x10⁹ Pa to 1x10⁶ Pa, or from 1x10⁹ Pa to 1x10⁷ Pa.

The polymers in the polymer compositions of this invention are preferably added to hair styling compositions to provide a total polymer concentration of from 0.1 to 15%, more preferably from 1 to 10%, and most preferably from 4 to 7%, based on the total weight of the hair styling composition. Typically gels will have a polymer concentration of from 0.5% to 4%, preferably 1% to 2%, and sprays will have a concentration of from 4% to 7%.

Hair styling compositions comprising the polymer compositions of this invention are applied to wet or dry hair by spraying or by rubbing onto the hair manually. The treated hair is then mechanically fixed in the desired configuration using, for example, any of a variety of hair styling implements such as, for example, combs, brushes, rollers, or curlers. When applied to wet hair, after application the hair may be dried using ambient air, electric, or hot air drying, before, during, or after styling. In some embodiments, hair is fixed in the desired configuration before hair styling composition is applied to the hair. In some embodiments, hair is fixed in the desired configuration after hair styling composition is applied to the hair.

The polymer compositions that are useful in hair styling compositions are soluble in the hair styling composition "as is" or upon neutralization of some or all of the acid groups contained in the polymer composition. The acidic groups in the polymer mixture of this invention, such as carboxylic acid groups, may be neutralized by conventional techniques with at least one base to dissolve the polymer in the hair styling composition. Bases that will neutralize the polymer mixture may be selected from one or more amines, alkali or alkaline earth metal hydroxides, and ammonium hydroxide. Suitable amine neutralizers include, for example, 2-amino-2-methyl-1,3-propanediol, 2-amino-2-methyl-1-propanol, N,N-dimethyl-2-amino-2-methyl-1-propanol, mono-isopropanolamine, triisopropanolamine, ethanolamine, triethanolamine, cyclohexylamine, fatty amines (such as, for example, stearyl dimethyl amine) and morpholine. Suitable alkali or alkaline earth metal hydroxides include, for example, sodium hydroxide and potassium hydroxide. Preferably, the neutralizer is selected from one or more of 2-amino-2-methyl-1,3-propanediol, 2-amino-2-methyl-1-propanol, N,N-dimethyl-2-amino-2-methyl-1-propanol, potassium hydroxide, triethanolamine, stearyl dimethyl amine, and triisopropanolamine.

In embodiments in which neutralizer is added to a composition of the present invention, the amount added is that amount needed to provide solubility of the polymer mixture in the hair styling composition and to ensure that the pH of the hair styling composition is cosmetically acceptable. In some embodiments, the amount of acid groups in the hair fixative resins that are neutralized, based on molar equivalents, is 5% or more; or 25% or more; or 50% or more. In some embodiments, the amount of acid groups in the hair fixative resins that are neutralized, based on molar equivalents, is 100% or less; or 75% or less. In some embodiments, no neutralizer is used.

In some embodiments, multistage polymers of the present invention have solution viscosity that falls within a desirable range. For example, the solution viscosity of a multistage polymer of the present invention may tested as follows. A sample of the multistage polymer is provided as an aqueous latex. To make a solution, ethanol, additional water, and water-soluble base could be added to the latex. The amount of water-soluble base could be chosen to neutralize 60 mole percent of the carboxylic acid groups on the multistage polymer. The amounts of ethanol and additional water could be chosen so that the resulting solution had 5% polymer solids and 55% ethanol, by weight based on the weight of the solution. In some embodiments the viscosity of such a solution will be 0.025 Pascal · seconds (Pa · sec) (25 centipoise) or less; or 0.015 Pa · sec (15 centipoise) or less. One appropriate method of measuring viscosity is with a Brookfield viscometer using ultra low adapter at 12 rpm.

In some embodiments, hair styling compositions are made that include one or more additional polymers in addition to at least one multistage polymer of the present invention. Such additional polymers include, for example, butyl acrylate/ethyl acrylate/methacrylic acid copolymers, polyvinylpyrrolidone, poly(vinyl pyrrolidone)/vinyl acetate copolymers, octylacrylamide/ acrylates/butylaminoethyl-methacrylate copolymers, vinylcaprolactam/vinyl-pyrrolidone/dimethylaminoethyl-methacrylate copolymers, methacryloyl ethyl-betaine/methacrylate copolymers, methacrylic acid/methacrylic ester copolymer, acrylates/hydroxyesters acrylates copolymer, methacrylic acid/acrylic acid ester copolymers. Additional hair fixative polymers useful for blending with the polymer compositions of this invention include, for example (by INCI name), ethyl ester of PVM/MA copolymer, butyl ester of PVM/MA copolymer, vinyl acetate/crotonic acid copolymer, vinyl acetate/crotonic acid/vinyl neodecanoate, VA/butyl maleate/isobornyl acrylate copolymer, acrylates copolymer, sulfonated polyester such as diglycol/CHDM/isophthalates/SIP copolymer, acrylates copolymer, acrylates terpolymer methacrylates/acrylates copolymer/amine salt, AMP-acrylates/diacetone-acrylamide copolymer, AMPD-acrylates/diacetone-acrylamide copolymer, acrylates/t-butylacrylamide copolymer, acrylates/methacrylate polymers, acrylates/acrylamide copolymer, PVP/vinyl caprolactam/DMAPA acrylates copolymer, polyvinylcaprolactam, isobutylene/ethylmaleimide/hydroxyethylmaleimide copolymer, acrylates/C1-2 succinates/hydroxyacrylates copolymer, carboxylated polyurethane such as polyurethane-1, polyurethane-6.

In some embodiments, for example embodiments in which a hair styling composition is made in the form of a gel, mousse, lotion, pomade, serum, or other form that is applied to hair by means other than spraying, additional polymers may include, for example, acrylates copolymer, acrylates/Hydroxyesters acrylates copolymer, acrylates C1-2 succinates/hydroxyacrylates copolymer, allyl stearate/vinyl acetate(VA) copolymer, AMP acrylate/diacetoneacrylamide copolymer, ethyl ester of PVM/MA copolymer, Butyl ester of PVM/MA copolymer, Isopropyl ester of PVM/MA copolymer, Octylacrylamide/acrylate/butylaminoethyl Methacrylate copolymer, phthalic anhydride/glycerin/glycidyl decanoate copolymer, polybutylene terephthalate, polyethylacrylate, polyethylene, polyvinyl acetate, polyvinyl butyral, polyvinyl methylether, polyvinylpyrolidinone (PVP), PVP/VA, PVP/dimethylaminoethylmethacrylate copolymer, PVP/eicosene copolymer, PVP/ethyl ethacrylate/methacrylic acid copolymer, PVP/hexadecane copolymer, PVP/VA itaconic acid polymer, sodium acrylate/vinyl alcohol copolymer, starch diethylaminoethyl ether, stearylvinyl ether/maleic anhydride copolymer, VA/crotonate copolymer, VA/crotonic acid copolymer, VA/crotonic acid/methacryloxybenzophenone-1 copolymer, VA/crotonic acid/vinyl neodecanoate copolymer, isobutylene/ethylmaleimide/hydroxyethylmaleimide copolymer, PVP/DMAPA acrylates copolymer, polyimide-1, Polyquatemium-4, polyquaternium-11, PQ-7, PQ-39, PQ-2, PQ-10, PQ-16, PQ-46, PQ-28, PQ-55, PQ-68, PVP/dimethylaminoethyl Methacrylate copolymer, Guar hydroxypropyl trimonium chloride, vinyl caprolactam/PVP/dimethyl aminoethyl Methacrylate copolymer, PVP and Dimethicone, PQ-28 and Dimethicone.

Some further examples of additional polymers that may be used in some embodiments in addition to at least one multistage polymer of the present invention include Polyurethane-14 (and) AMP-Acrylates Copolymer, Acrylates/Diacetoneacrylamide copolymer, Aminoethylpropanediol-Acrylates/Acrylamide copolymer, Aminoethylpropanodiol-AMPD-Acrylates/Diacetoneacrylamide Copolymer, AMP-Acrylates/C1-8 Alkyl Acrylates/C1-8 Alkyl Acrylamide Copolymer, AMP-Acrylates Copolymer, and AMP-Acrylates/Diacetoneacrylamide Copolymer. In some embodiments, at least one multistage polymer of the present invention is blended with at least one octylacrylamide/acrylates/butlyaminoethyl-methacrylate copolymer.

Also, in some embodiments, compositions may be used that contain, in addition to the multistage polymer of the present invention, one or more amphoteric polymers. Among embodiments in which amphoteric polymers are used, the composition may or may not contain one or more of the other polymers described herein above as appropriate for use in addition to the multistage polymer. An amphoteric polymer is a polymer that has at least one anionic group covalently attached to the polymer and at least one cationic group covalently attached to the polymer. An anionic group is a group that, when the polymer is in an aqueous composition, there is a range of pH values in which that group exists as an anion. A cationic group is a group that, when the polymer is in an aqueous composition, there is a range of pH values (which may be the same as, overlapping, or distinct from, the range of pH values over which the anionic group exists as an anion) in which that group exists as a cation. Some suitable amphoteric polymers are, for example, octylacrylamide/ acrylates/butylaminoethyl-methacrylate copolymers, vinylcaprolactam/vinyl-pyrrolidone/dimethylaminoethyl-methacrylate copolymers, methacryloyl ethyl-betaine/methacrylate copolymers, AMP-acrylates/diacetone-acrylamide copolymer, AMPD-acrylates/diacetone-acrylamide copolymer, acrylates/t-butylacrylamide copolymer, acrylates/methacrylate polymers, acrylates/acrylamide copolymer, PVP/vinyl caprolactam/DMAPA acrylates copolymer, polyvinylcaprolactam, isobutylene/ethylmaleimide/hydroxyethylmaleimide copolymer. In some embodiments, one or more octylacrylamide/ acrylates/butylaminoethyl-methacrylate copolymer is used.

In some embodiments, additional polymers are used that have solution viscosity the same as, or lower than, the solution viscosity of multistage polymers of the present invention, measured as described herein above (i.e., in solution that is 5% polymer solids, by weight based on the weight of solution, in a solvent that is a mixture of ethanol and water, with weight ratio of ethanol to water of 55 to 40). In some embodiments, additional polymer is chosen so that the solution viscosity of the blend, measured as described herein above, is lower than the solution viscosity of the additional polymer alone and is lower than the solution viscosity of the multistage polymer of the present invention alone.

In addition to the polymer compositions of this invention, hair styling compositions may contain any other ingredient used in cosmetics such as, for example, perfumes, dyestuffs which can color the hair styling composition itself or hair fibers, preservatives, sequestering agents, thickeners, silicones, softeners, foam synergistic agents, foam stabilizers, sun filters, peptizing agents, conditioning agents, shine agents, proteins, herbals, botanicals, neutralizers, plasticizers, and anionic, non-ionic, cationic, or amphoteric surfactants, or mixtures thereof.

One or more surfactants may be added to the hair styling composition, typically to reduce the surface tension of the composition. When surfactants are present in the hair styling composition, they are preferably present at a concentration of from 0.001 to 1%, based on the total weight of the composition.

One or more plasticizers may be added to the hair styling composition of the present invention. When plasticizers are present in the hair styling composition, they are preferably present at a concentration of from 0.001 to 1%, based on the total weight of the composition. The plasticizers that may be used in the hair styling composition include, for example, dimethicone copolyol, dimethicone, phenyltrimethicones, trialkylcitrates, cyclomethicone, disiloxane, and others that are known and typically used in the art.

Hair styling compositions comprising the polymer compositions of this invention are preferably solutions in which the solvent is any cosmetically acceptable solvent. Water or other solvents may be used alone or in mixtures. In some embodiments, the solvent is water or a mixture of water and a water-miscible solvent other than water (such as, for example, one or more alcohol such as, for example, ethanol). In some embodiments, the solvent has 10% water or less, by weight based on the weight of the solvent; or 5% water or less; or 1% water or less. Such solvents may be present in proportions of up to 99.9 percent of the hair styling composition by weight based on the weight of the hair styling composition.

In a hair styling composition using an aerosol spray, one or more propellants are used. Preferably the propellants are used at a total concentration of from 10 to 70%, more preferably from 30 to 60%, based on the total weight of the hair styling composition. Suitable propellants include, for example, hydrocarbons such as propane, n-butane, isobutane, and pentane; ethers such as dimethyl ether; fluorocarbons (such as, for example, difluoroethane), and mixtures thereof. Preferred propellants are selected from one or more of dimethyl ether, 1,1-difluoroethane, propane, n-butane and isobutane. These propellants are available commercially.

Preservatives may be used in the hair styling composition including, for example, one or more of isothiazolinones, iodopropynylbutyl carbamate, benzyl alcohol, imidazolidinylurea, benzoic acid, methylisothiazolinones, alkyl parabens, and mixtures thereof.

One or more thickeners may be desirable, for example in a hair styling composition that is applied to the hair in the form of a gel, mousse, lotion, pomade, serum, or other form that is applied to hair by means other than spraying. Suitable thickeners include, for example, polycarboxylic acid thickeners such as acrylates/steareth-20 methacrylate copolymer, acrylates copolymer, or acrylates C₁₀₋₃₀ alkyl acrylate crosspolymer; carbomers, hydroxyethyl cellulose, PVM/MA decadiene crosspolymer, steareth-10 allyl ether/acrylate copolymer, hydrophobically modified polyethoxylated urethane thickeners, starch-based thickeners, and polyamide thickeners. Additional suitable thickeners include, for example, acrylic rheology modifiers, including, for example, Acrylates/Steareth-20 Methacrylate Copolymer, Acrylates/Beheneth-25 Methacrylate Copolymer, Acrylates Copolymer, PEG-150/Decyl Alcohol/SMDI Copolymer, PEG-150/Stearyl Alcohol/SMDI Copolymer, PEG-150/Distearate, Acrylates/Steareth-20 Methacrylate Crosspolymer, and Acrylates/Vinyl Neodecanoate Crosspolymer. Mixtures of suitable thickeners are also suitable. The thickeners, when used, preferably are present at a total concentration of from 0.001 to 5%, based on the total weight of the composition.

In some embodiments, it is contemplated that the use of one or more acrylic rheology modifier may, in some cases, further improve the performance of the composition. For example, the use of one or more acrylic rheology modifier may, in some cases, improve the stiffness, humidity resistance, or both, of the hair styling composition after it has been applied to hair. It is contemplated that the type and amount of rheology modifier, when used, will be chosen so that the desirable viscosity of the hair styling composition is maintained (for example, compositions designed to be sprayed onto hair normally have a lower viscosity than compositions designed to be applied to hair as a gel).

Independently or additionally, the use of one or more acrylic rheology modifier may, in some cases, improve the properties of a hair styling composition as the hair styling composition exists before it is applied to hair. For example, inclusion of one or more acrylic rheology modifier may, in some cases, improve the foam density or foam stability or both of a mousse.

Other additives, such as those commonly used by those skilled in the art, may be added to the hair styling composition. The other additives used in the hair styling composition will depend upon the type of hair styling composition desired. Other additives include, for example, one or more of; moisturizers (such as glycerine, hydrolyzed silk protein, and hydrolyzed wheat protein); conditioning agents such as panthenol; conditioning agents (U.S. Patent No. 5,164,177 may be consulted for further general and specific details on suitable conditioning agents); emulsifiers; antistatic aids; extracts; proteins; vitamins; colorants; UV protectors; fragrances, and corrosion inhibitors. Such other additives typically comprise from 0.005 to 5%, and more preferably from 0.01 to 1%, of the hair styling composition.

Additives, including surfactants, solvents, other preservatives, and thickeners, that may be suitable in the hair styling compositions may be found in the International Cosmetic Ingredients Dictionary, 9th Edition, 2002, published by the Cosmetics Toiletries Fragrances Association (CFTA), Washington D.C.

In addition to use in hair styling compositions, the compositions of the present invention are also contemplated for use in other compositions useful in hair care, skin care, cosmetics, or other related uses. For example, the compositions of the present invention are contemplated for use in one or more of hair mask, hair conditioner, hair shampoo, eye mascara, body wash, skin mask, skin lotion, color cosmetics, make-up, lipstick, or other related uses.

It is to be understood that for purposes of the present specification and claims that the range and ratio limits recited herein can be combined. For example, if ranges of 60 to 120 and 80 to 110 are recited for a particular parameter, it is understood that the ranges of 60 to 110 and 80 to 120 are also contemplated. As a further, independent, example, if a particular parameter is disclosed to have suitable minima of 1, 2, and 3, and if that parameter is disclosed to have suitable maxima of 9 and 10, then all the following ranges are contemplated: 1 to 9, 1 to 10, 2 to 9, 2 to 10, 3 to 9, and 3 to 10.

### EXAMPLES

### Example 1: Multistage polymer P1

To a three liter, four-neck round bottom flask quipped with overhead stirrer, condenser, nitrogen adapter and a thermocouple was added 430 parts water, 10.9 parts of benzoic acid, and 19.2 parts of Rhodafac RS-610A (available from Rhodia). Separately, a stage-1 monomer emulsion was prepared by mixing 183 parts of water, 6.4 parts of Rhodafac RS-610A, 80 parts of butyl acrylate (BA), 200 parts of ethyl acrylate (EA), 60 parts of hydroxyethyl methacrylate (HEMA), 60 parts of methacrylic acid (MAA), and 4 parts of n-dodecyl mercaptan (n-DDM). With the nitrogen turned on, the reactor and contents at 85 C, 42 parts of the above stage-1 monomer emulsion was charged with stirring, followed by an initiator solution of 1 part of sodium persulfate dissolved in 15 parts of water. The remaining monomer emulsion was then fed over 48 minutes, while maintaining a temperature of 85°C. A cofeed initiator solution containing 1 part of sodium persulfate and 73 parts of water was gradually added simultaneously with this monomer feed as well as stage 2 monomer feed as described later. After stage-1 monomer was completely fed, stage-2 monomer was prepared by mixing 270 parts of water, 9.6 parts of Rhodafac RS-610A, 150 parts of BA, 282 parts of methyl methacrylate(MMA), 60 parts of HEMA, 108 parts of MAA, and 1.8 parts of n-DDM. The stage-2 monomer emulsion was fed over 72 minutes, while maintaining a temperature of 85C.

After the monomer emulsion and initiator feeds were complete, the reaction mixture was "chased" with a ferrous sulfate, t-butyl hydroperoxide, ammonium persulfate, D-isoascorbic acid combination to reduce residual monomer levels. The reaction mixture was then cooled to room temperature and filtered. The emulsion polymer prepared had solids of 47%.

### Example 2: Preparation of Comparative Polymers CB, CC, and CD

Using the methods of Example 1, a multistage polymer (Comparative Polymer CB) was prepared with the same composition in each individual stage as in Polymer P1, but with the amounts of stage-1 and stage-2 adjusted so that the weight ratio of the second stage polymer to the first stage polymer was 40:60. The amount of n-DDM in the first stage was 1% by weight based on the weight of monomers in the first stage. The amount of n-DDM in the second stage was 0.6% by weight based on the weight of monomers in the second stage.

A film of Comparative Polymer CB was made by drying at 60°C under vacuum. That film of Comparative Polymer CB was thoroughly dried by heating to 140°C in the DSC instrument in a vented pan, cooled in the DSC instrument, and then was measured by DSC during a second heating. Comparative Polymer CB showed glass transition temperatures of 43°C and 97°C.

Comparative Polymer CC was Resyn^{™} 28-2930, from National Starch.

Comparative Polymer CD was Amphomer^{™} LV-71 from National Starch.

### Example 3: Preparation of Multistage Polymer A

Using the methods of Example 1, a multistage polymer (Polymer A) was prepared with the same composition in each individual stage as in Polymer P1, but with the amounts of stage-1 and stage-2 adjusted so that the weight ratio of the second stage polymer to the first stage polymer was 60:40. The amount of n-DDM in the first stage was 1% by weight based on the weight of monomers in the first stage. The amount of n-DDM in the second stage was 0.3% by weight based on the weight of monomers in the second stage.

A film of Polymer A was made by drying at 60°C under vacuum. That film of Polymer A was thoroughly dried by heating to 140°C in the DSC instrument in a vented pan, cooled in the DSC instrument, and then was measured by DSC during a second heating. Polymer A showed glass transition temperatures of 34°C and 89°C.

Additionally, a sample of the polymer produced by stage-1 was made, and a film thereof was made (in the absence of any stage-2 polymer), dried, and tested by DSC using the same method used for the film of Polymer A. That sample showed glass transition temperature of 30°C.

Additionally, a sample of the polymer produced by stage-2 was made in the absence of stage-1 polymer. A film thereof was made , dried, and tested by DSC using the same method used for the film of Polymer A. That sample showed glass transition temperature of 93°C.

### Example 4: Tg versus Relative Humidity

Samples of Polymer A and Comparative Polymers CB and CC were dried at 60°C under vacuum. Each sample was conditioned at a certain relative humidity ("RH") and then sealed into a pan. The sample, in the sealed pan, was then tested for thermal transitions by differential scanning calorimetry during a first heating. In the case of Comparative Polymers CB and CC, only one thermal transition was detected in each measurement, while Polymer A showed two thermal transitions in each measurement. The results were as follows. Thermal transition temperature results are reported in °C.

| Polymer | RH (%) | first transition temperature | second transition temperature |
|---|---|---|---|
| A | 0 | 26 | 50 |
| A | 33 | 18 | 51 |
| A | 58 | 8.5 | 50 |
| A | 75 | -3.0 | 47 |
| CB | 0 | 39 | none |
| CB | 32 | 23 | none |
| CB | 57 | 9.5 | none |
| CB | 78 | -3.3 | none |
| CC | 0 | 47 | none |
| CC | 33 | 46 | none |
| CC | 58 | 21 | none |
| CC | 75 | 18 | none |

### Example 5: High Humidity Curl Retention

European brown virgin hair swatches, 20.3 cm (8 inch) long and 2.0±0.1 grams, obtained from International Hair Importer, New York were used. Hair was washed in mild shampoo and curled wet onto a 22 millimeter ("mm")x70 mm curler and held in place with a bobby pin. The curled tresses were allowed to air dry on a lab bench overnight, and dried in 45°C oven for 20 minutes prior to treatment.

Solutions were prepared as described herein above for preparing samples for measurement of solution viscosity. In each solution, polymer was 60 mole percent neutralized, polymer solids was 5% by weight, and the ethanol content was 55% by weight, based on the weight of the solution.

The curled tresses were uniformly sprayed with the solution twice in the front and twice on the back from a distance of 15.2 cm (6 inch) distance with the hair sprays. The spray device delivered 190 µL (microliters) of formulation with each compression. The spray device product was "Euromist Classic", manufactured by SequistPerfect, Cary, IL. The curled, treated tresses were dried for 1 hour in a controlled environment at 22.5 °C and 55% relative humidity. The curler was removed carefully without disturbing the tress. Curls were suspended by clips in a humidity chamber at 90% RH, 25°C. Initial curl length was recorded. The length of the curled tresses was again recorded after 4 hours. Curl retention is determined as [(L(0)-L(t))/L(0)-L(i))x100] where L(0) is fully extended curl length, L(i) is initial curl length and L(t) is curl length after 4 hours. Hair treated with polymer A showed curl retention of 75%, while hair treated with comparative polymer CB showed curl retention of 54%.

### Example 6: Tensile Testing of Curled Tresses

Curled hair swatches were prepared following the procedure of Example 5. The curled tress was placed in Dia-Stron™ miniature tensile tester, model MTT160 instrument (Dia-Stron Limited, Unit 9 Focus 303 Business Centre, Andover, Hampshire SP10 5NY UK, or 390 Reed Road, Broomall, Pa 19008, USA) and the work to compress the curl to 25% of its initial diameter was measured. The compression was repeated 2-5 times for each tress. Measurements were made at room temperature and 55% relative humidity. The stress versus strain curve was recorded during the cycles of curl compression. Peak force and modulus (the slope of the stress versus strain) was recorded and calculated to characterize polymer film hardness. The higher the value indicates stiffer and crunchier film on hair.

Modulus values were 8.9 for polymer A and 5 for comparative polymer CB.

In a separate measurement, modulus values were 8.6 for polymer A and 3.8 for comparative polymer CC.

Peak force values were 244 grams force (gmf) for polymer A and 156 gmf for comparative polymer CB.

In a separate measurement, peak force values were 209 gmf for polymer A and 109 gmf for comparative polymer CC.

### Example 7: Solution Viscosity

Additional aqueous latex multistage polymers were made using the methods of Example 1, with the amounts of n-DDM shown (as weight % based on the weight of monomers in that stage) and with the weight ratios of second stage to first stage shown.

The latex forms of the various polymers were each treated as follows to make a solution: ethanol was added to the latex, followed by additional water, followed by neutralizer. The amount of neutralizer was chosen to neutralize 60 mole percent of the acid groups of the polymer. The amounts of ethanol and additional water were chosen to give solutions of 5% polymer solids, by weight based on the weight of solution, with a solvent that was ethanol and water, with weight ratio of ethanol to water of 55 to 40. The viscosity of each solution was then measured with a Brookfield viscometer using ultra low adapter at 12 rpm. The results were as follows. Viscosity is reported in milliPascal*seconds (mPa*s), which is numerically the same as centipoise.

| | n-DDM (%) | | Viscosity, mPa*s | | | |
|---|---|---|---|---|---|---|
| Example No. | first stage | second stage | 55/45⁽¹⁾ | 60/40⁽¹⁾ | 63/35⁽¹⁾ | 70/30⁽¹⁾ |
| 7-1 | 0.6 | 0.15 | 22.5 | 15.2 | NM⁽²⁾ | NM⁽²⁾ |
| 7-2 | 0.6 | 0.3 | 17.3 | 12.7 | 18.0 | 19.0 |
| 7-3 | 0.6 | 0.5 | 13.4 | 10.7 | 13.5 | 14.0 |
| 7-4 | 1 | 0.3 | 15.3 | 11.8 | 16.5 | 16.6 |
| 7-5 | 1 | 0.5 | 11.5 | 9.5⁽³⁾ | 12.8 | 12.7 |
| 7-6 | 1.25 | 0.15 | 19.0 | 13.2 | 21.2 | 22.8 |
| 7-7 | 1.25 | 0.3 | 14.7 | 11.2 | 16.0 | 16.7 |
| 7-8 | 1.25 | 0.5 | 10.9 | 10.5 | 10.9 | 12.7 |

| | | | | | | |
|---|---|---|---|---|---|---|
| Note (1): weight ratio of second stage polymer to first stage polymer Note (2): not made Note (3): Example 7-5 with stage ratio of 60/40 is the same as polymer A. | | | | | | |

### Example 8: Dynamic Mechanical Analysis

For each polymer, dry films were prepared. The samples were tested on the Rheometrics Mechanical Spectrometer (RMS-800) in a Dynamic Temperature Ramp Mode using 8 mm parallel plates. The plates were zeroed at the maximum scan temperature. The samples were placed the lower plate, then the upper plate was brought into contact with the sample with sufficient force so the soft samples filled the gap between the two plates. All scans were performed with an applied strain of 0.05%, and an applied frequency of 6.28 rad/s at a cooling rate of 2 °C/min, from approximately 180°C to approximately 2°C. The dynamic storage (G') and loss (G") moduli were recorded as a function of temperature as well as the loss tangent (tan delta).

Polymer A was compared to comparative polymers CC and CD

In tandelta results, polymer A showed two peaks, at 64°C and 125°C, while comparative polymer CD showed a single peak at 167°C. At 25°C, polymer A showed G' of 2.2 X 10⁹ dyn/cm², comparative polymer CC showed G' of 1.0 X 10⁹ dyn/cm², and comparative polymer CD showed G' of 6.5 X 10⁸ dyn/cm².

### Example 9: Bouncing Curl Test

Hair swatches were prepared as in Example 5. The treated curls are mounted on a bouncing device. The initial lengths of the curls were measured and recorded. The hair was "bounced" at 70 cycles/minute. The lengths of the curls were measured after 6-8 hour of bouncing test. The percent of curl retention is calculated to characterize the style retention and durability.

The stress versus strain curve was recorded during the cycles of curl compression by Dia-Stron™ Curl Compression test. The slope of the stress versus strain curve is calculated as modulus; the retention of modulus value between first compression and second compression is calculated as modulus retention to characterize stiffness durability of film on hair. The results were as follows:

| Polymer | Style Retention (%) | Modulus Retention (%) |
|---|---|---|
| A | 82 | 76 |
| CD | 80 | 72 |
| CC | 73 | not tested |

### Example 10: Coagulation

Each 3L batch coagulation began by adding 600g of the preheated 30% solids latex to 1200g of the dilute calcium chloride over approximately 1 minute. The stirrer was fixed at about 500 rpm throughout the latex addition. After the latex addition, a 1 or 2 minute wait ensued. The flow aid, generally 4.0, 6.0, or 10.0% solids (based on total latex solids) was then added to the slurry over a few seconds. The flow aid was a latex of an acrylic polymer with Tg of at least 70°C and mean particle size of 50 to 300 nm. Approximately 0.5 - 1.0 minutes after the flow aid addition, an optional 15 - 45g of 0.2 g/ml CaCl₂ were added. After the stirring speed was reduced to 400 rpm, the heating jacket was turned on high to cook the slurry to the desired final temperature, 75 - 90°C. Once the slurry reached the desired cook temperature, it was stirred with a magnetic stirrer and allowed to cool to at least 60°C before being vacuum filtered and washed 7 to 1 based on primary latex solids. Finally, the wet cake was dried overnight at about 45°C in a vacuum oven.

Attempts to coagulate Polymer B using calcium chloride indicated that the polymer fused together in the days following coagulation, unless a flow aid was used. Polymer A, on the other hand, was easily coagulated and remained flowable over the course of the study regardless of whether flow aid was used or not. Coagulation of Polymer A produced particles from the 80°C tank with an average size of 227.92 µm, 0% under 47 µm, and 1.93% over 600 µm, with a span of 1.391. "Span" is determined by the formula (D90-D10)/D50, where D 10 is the diameter where 10% of the particles (by weight, based on the total weight of particles) are less than D10, D50 is the diameter where 50% of the particles (by weight, based on the total weight of particles) are less than D50, and D90 is the diameter where 90% of the particles (by weight, based on the total weight of particles) are less than D90.

### Example 11: Spray Drying

A tower spray dryer equipped with a spray nozzle was used at the following operating conditions to provide an estimated powder temperature of approximately 49°C: nozzle pressure was 1550 psi, and emulsion feed rate was 1806 parts per hour. No flow aid was used. A free flowing powder having a mean powder particle diameter of 200 micrometers was produced. A compaction-free powder was evident, because the resulting powder did not stick together in a solids mass when hand-squeezed.

### Example 12: Blends with Amphoteric Polymer

Solutions could be made in solvent that is mixed ethanol and water, with ratio of ethanol to water of 1:375:1 by weight. Each solution could have 5% polymer solids by weight based on the weight of the solution. The viscosity of each solution could be measured as in Example 7.

Each solution could have an octylacrylamide/ acrylates/butylaminoethyl-methacrylate copolymer ("Amphomer") and a second polymer ("2ndP"). Blends that could be made, and the resulting viscosities that would be measured, would be as follows.

| | Viscosity, mPa*s | | |
|---|---|---|---|
| | Second Polymer | | |
| Weight Ratio: Amphomer / 2ndP | Stage-2 | Stage-1 | Polymer A |
| 0/100 | 13.6 | 10.3 | 10.1 |
| 20/80 | 14.3 | 9.95 | 9.7 |
| 50/50 | 14.2 | 11.5 | 9.7 |
| 80/20 | 15.5 | 12.5 | 11.3 |
| 100/0 | 16.7 | 16.7 | 16.7 |

The viscosity value for each blend of Amphomer with Polymer A is significantly less than the weighted average of the viscosities that would be measured for each polymer alone. The weighted average is VWA = R*VAM + (1-R)*VPA, where R is the weight ratio of Amphomer to Polymer A, VAM is the viscosity of the solution of Amphomer alone, and VPA is the viscosity of the solution of Polymer A alone.

## Claims

1. A multistage polymer that comprises
(a) at least one soft polymer having glass transition temperature of 40°C or lower, and
(b) at least one hard polymer having glass transition temperature higher than 40°C, wherein the glass transition temperature of said hard polymer is at least 10°C higher than the glass transition temperature of said soft polymer, wherein said hard polymer comprises monomer units, by weight based on the weight of said hard polymer, of
(i) 5% to 50% unsubstituted alkyl esters of acrylic acid,
(ii) 10% to 75% unsubstituted alkyl esters of methacrylic acid,
(iii) 2% to 50% hydroxyalkyl esters of acrylic acid or of methacrylic acid, and
(iv) 10% to 30% acid functional monomer units
wherein the weight ratio of said hard polymer to said soft polymer is from 1.01:1 to 100:1, and
wherein said multistage polymer, after exposure to liquid water followed by drying at temperatures below 100°C, shows maximum thermal transition temperature in an atmosphere of 0% relative humidity that differs by 20°C or less from the maximum thermal transition temperature in an atmosphere of 75% relative humidity.

2. The multistage polymer of claim 1, wherein said hard polymer is made by polymerizing a mixture that comprises at least one monomer and at least one chain transfer agent, wherein the amount of said chain transfer agent is 0.5% or less by weight based on the weight of all monomers in said mixture.

3. An aqueous polymer latex comprising the multistage polymer of claim 1.

4. A composition comprising
(i) 0 to 15% by weight volatile compounds, based on the weight of said composition, and
(ii) at least one powder comprising at least one multistage polymer of claim 1.

5. The composition of claim 4, wherein said powder is produced by providing an aqueous latex comprising said multistage polymer and then isolating said multistage polymer by a process comprising spray drying or coagulation.

6. A solution comprising at least one solvent and at least one multistage polymer of claim 1.

7. The solution of claim 6, wherein said solvent comprises water or at least one water-soluble alcohol or a mixture of water and at least one water-soluble alcohol.

8. The solution of claim 6, wherein said solution is anhydrous.

9. The solution of claim 6, wherein said solution further comprises at least one amphoteric polymer.

10. A method for styling hair comprising the steps of
(A) placing said hair in a desired configuration and
(B) applying the solution of claim 7 to said hair.

11. The polymer of claim 1, wherein said soft polymer comprises 10% or more acid functional monomer units, by weight based on the weight of said soft polymer.

12. The polymer of claim 1, wherein said soft polymer comprises 5% or more acid functional monomer units, by weight based on the weight of said soft polymer.

13. The polymer of claim 1, wherein said soft polymer comprises 10% or more monomer units of hydroxyalkyl ester of acrylic acid or methacrylic acid, by weight based on the weight of said soft polymer.

14. The polymer of claim 1, wherein said soft polymer additionally comprises 5% or more monomer units of hydroxyalkyl ester of acrylic acid or methacrylic acid, by weight based on the weight of said soft polymer.

15. The polymer of claim 1, wherein said hard polymer additionally comprises 5 to 25% or more monomer units of hydroxyalkyl ester of acrylic acid or methacrylic acid, by weight based on the weight of said hard polymer.

## Patentansprüche

1. Mehrstufenpolymer, welches umfaßt
(a) mindestens ein Softpolymer mit einer Glasübergangstemperatur von 40°C oder weniger, und
(b) mindestens ein Hartpolymer mit einer Glasübergangstemperatur von höher als 40°C, wobei die Glasübergangstemperatur des Hartpolymers mindestens 10°C höher als die Glasübergangstemperatur des Softpolymers ist, wobei das Hartpolymer Monomereinheiten, bezogen auf das Gewicht, basierend auf dem Gewicht des Hartpolymers, von
(i) 5% bis 50% unsubstituierten Alkylestern von Acrylsäure,
(ii) 10% bis 75% unsubstituierten Alkylestern von Methacrylsäure,
(iii) 2% bis 50% Hydroxyalkylestern von Acrylsäure oder von Methacrylsäure, und
(iv) 10% bis 30% säurefunktionalen Monomereinheiten umfaßt,
wobei das Gewichtsverhältnis des Hartpolymers zu dem Softpolymer von 1,01:1 bis 100:1 ist, und
wobei das Mehrstufenpolymer nach Aussetzung an flüssiges Wasser, gefolgt von Trocknen bei Temperaturen unterhalb 100°C, eine maximale Wärmeübergangstemperatur in einer Atmosphäre von 0% relativer Feuchtigkeit zeigt, die sich um 20°C oder weniger von der maximalen Wärmeübergangstemperatur in einer Atmosphäre von 75% relativer Feuchtigkeit unterscheidet.

2. Mehrstufenpolymer gemäß Anspruch 1, wobei das Hartpolymer durch Polymerisieren eines Gemisches hergestellt wird, welches mindestens ein Monomer und mindestens ein Kettenübertragungsmittel umfaßt, wobei die Menge des Kettenübertragungsmittels 0,5% oder weniger, bezogen auf das Gewicht, basierend auf dem Gewicht sämtlicher Monomere in dem Gemisch, beträgt.

3. Wäßriger Polymerlatex, umfassend das Mehrstufenpolymer gemäß Anspruch 1.

4. Zusammensetzung, umfassend
(i) 0 bis 15 Gew.-% flüchtige Verbindungen, bezogen auf das Gewicht der Zusammensetzung, und
(ii) mindestens ein Pulver, umfassend mindestens ein Mehrstufenpolymer gemäß Anspruch 1.

5. Zusammensetzung gemäß Anspruch 4, wobei das Pulver durch Bereitstellen eines wäßrigen Latex, umfassend das Mehrstufenpolymer, und anschließendes Isolieren des Mehrstufenpolymer durch ein Verfahren, umfassend Sprühtrocknen oder Koagulation, hergestellt wird.

6. Lösung, umfassend mindestens ein Lösungsmittel und mindestens ein Mehrstufenpolymer gemäß Anspruch 1.

7. Lösung gemäß Anspruch 6, wobei das Lösungsmittel Wasser oder mindestens einen wasserlöslichen Alkohol oder ein Gemisch von Wasser und mindestens einem wasserlöslichen Alkohol, umfaßt.

8. Lösung gemäß Anspruch 6, wobei die Lösung wasserfrei ist.

9. Lösung gemäß Anspruch 6, wobei die Lösung weiter mindestens ein amphoteres Polymer umfaßt.

10. Verfahren zum Hairstyling, umfassend die Schritte
(A) Anordnen des Haares in einer gewünschten Konfiguration, und
(B) Aufbringen der Lösung gemäß Anspruch 7 auf das Haar.

11. Polymer gemäß Anspruch 1, wobei das Softpolymer 10% oder mehr säurefunktionale Monomereinheiten, bezogen auf das Gewicht, basierend auf dem Gewicht des Softpolymers, umfaßt.

12. Polymer gemäß Anspruch 1, wobei das Softpolymer 5% oder mehr säurefunktionale Monomereinheiten, bezogen auf das Gewicht, basierend auf dem Gewicht des Softpolymers, umfaßt.

13. Polymer gemäß Anspruch 1, wobei das Softpolymer 10% oder mehr Monomereinheiten von Hydroxyalkylester von Acrylsäure oder Methacrylsäure, bezogen auf das Gewicht, basierend auf dem Gewicht des Softpolymers, umfaßt.

14. Polymer gemäß Anspruch 1, wobei das Softpolymer zusätzlich 5% oder mehr Monomereinheiten von Hydroxyalkylester von Acrylsäure oder Methacrylsäure, bezogen auf das Gewicht, basierend auf dem Gewicht des Softpolymers, umfaßt.

15. Polymer gemäß Anspruch 1, wobei das Hartpolymer zusätzlich 5 bis 25% oder mehr Monomereinheiten von Hydroxyalkylester von Acrylsäure oder Methacrylsäure, bezogen auf das Gewicht, basierend auf dem Gewicht des Hartpolymers, umfaßt.

## Revendications

1. Polymère multi-étapes qui comprend
(a) au moins un polymère mou présentant une température de transition vitreuse de 40°C ou inférieure, et
(b) au moins un polymère dur présentant une température de transition vitreuse supérieure à 40°C, dans lequel la température de transition vitreuse dudit polymère dur est au moins 10°C supérieure à la température de transition vitreuse dudit polymère mou, dans lequel ledit polymère dur comprend des unités monomères, en masse rapportés au poids dudit polymère dur, de
(i) 5 % à 50 % d'esters alkyliques non substitués d'acide acrylique,
(ii) 10 % à 75 % d'esters alkyliques non substitués d'acide méthacrylique,
(iii) 2 % à 50 % d'esters hydroxyalkyliques d'acide acrylique ou d'acide méthacrylique, et
(iv) 10 % à 30 % d'unités monomères acides fonctionnels
dans lequel le rapport massique dudit polymère dur audit polymère mou est de 1,01 : 1 à 100 : 1, et
dans lequel ledit polymère multi-étapes, présente, après exposition à de l'eau liquide suivie par un séchage à des températures inférieures à 100°C, une température de transition thermique maximale dans une atmosphère d'humidité relative de 0 % qui diffère de 20°C ou moins de la température de transition thermique maximale dans une atmosphère de 75 % d'humidité relative.

2. Polymère multi-étapes selon la revendication 1, dans lequel ledit polymère dur est fabriqué par polymérisation d'un mélange qui comprend au moins un monomère et au moins un agent de transfert de chaîne, dans lequel la quantité dudit agent de transfert de chaîne est de 0,5 % ou inférieure en poids, rapporté à la masse de tous les monomères dans ledit mélange.

3. Latex polymère aqueux comprenant le polymère multi-étapes selon la revendication 1.

4. Composition comprenant
(i) de 0 à 15 % en poids de composés volatils, rapportés à la masse de ladite composition, et
(ii) au moins une poudre comprenant au moins un polymère multi-étapes selon la revendication 1.

5. Composition selon la revendication 4, dans laquelle ladite poudre est produite en fournissant un latex aqueux comprenant ledit polymère multi-étapes et en isolant ensuite ledit polymère multi-étapes par un procédé comprenant un séchage par pulvérisation ou une coagulation.

6. Solution comprenant au moins un solvant et au moins un polymère multi-étapes selon la revendication 1.

7. Solution selon la revendication 6, dans laquelle ledit solvant comprend de l'eau ou au moins un alcool soluble dans l'eau ou un mélange d'eau et d'au moins un alcool soluble dans l'eau.

8. Solution selon la revendication 6, dans laquelle ladite solution est anhydre.

9. Solution selon la revendication 6, dans laquelle ladite solution comprend de plus au moins un polymère amphotère.

10. Procédé pour le coiffage des cheveux comprenant les étapes
(A) de placement desdits cheveux dans une configuration souhaitée et
(B) d'application de la solution selon la revendication 7 sur lesdits cheveux.

11. Polymère selon la revendication 1, dans lequel ledit polymère mou comprend 10 % ou plus d'unités monomères acides fonctionnels, en masse rapportés à la masse dudit polymère mou.

12. Polymère selon la revendication 1, dans lequel ledit polymère mou comprend 5 % ou plus d'unités monomères acides fonctionnels, en masse rapportés à la masse dudit polymère mou.

13. Polymère selon la revendication 1, dans lequel ledit polymère mou comprend 10 % ou plus d'unités monomères d'ester hydroxyalkylique d'acide acrylique ou d'acide méthacrylique, en masse rapportés à la masse dudit polymère mou.

14. Polymère selon la revendication 1, dans lequel ledit polymère mou comprend en plus 5 % ou plus d'unités monomères d'ester hydroxyalkylique d'acide acrylique ou d'acide méthacrylique en masse rapportés à la masse dudit polymère mou.

15. Polymère selon la revendication 1, dans lequel ledit polymère dur comprend en plus de 5 à 25 % ou plus d'unités monomères d'ester hydroxyalkylique d'acide acrylique ou d'acide méthacrylique, en masse rapportés à la masse dudit polymère dur.
